# EUROPÄISCHE PATENTSCHRIFT

(11) **EP 0 597 382 B1**
(45) Veröffentlichungstag und Bekanntmachung des Hinweises auf die Patenterteilung: **17.07.1996**
(21) Anmeldenummer: 93117851.1
(22) Anmeldetag: 04.11.1993
(51) Int. Cl.: C07C 267/00, C07C 265/10, C08K 5/29, C08G 18/00

(54) **Neue Bis-(4-substituierte-2,6-diisopropyl-phenyl)-carbodiimide, ein Verfahren zu ihrer Herstellung und ihre Verwendung sowie die zu ihrer Herstellung verwendbaren 4-substituierten, 2,6-Diisopropyl-phenylisocyanate**
Bis-(4-substituted-2,6-diisopropylphenyl)-carbodiimides, process for their preparation and their use and the intermediates for their preparation 4-substituted-2,6-diisopropyl phenylisocyanate
Bis-(4-subsituées-2.6-diisopropylphényl)-carbodiimides, procédé pour leur préparation et leur utilisation et 4-substitutés-2,6-diisopropyl-phenylisocyanates comme intermédiaires pour leur préparation

(30) Priorität: 11.11.1992 DE 4238046
(43) Veröffentlichungstag der Anmeldung: 18.05.1994
(73) Patentinhaber: BASF Aktiengesellschaft, D-67063 Ludwigshafen (DE)
(72) Erfinder: Lehrich, Friedhelm, Dr., D-49448 Lemfoerde (DE); Pohl, Siegmund, D-67069 Ludwigshafen (DE); Bruchmann, Bernd, Dr., D-67069 Ludwigshafen (DE); Tesch, Helmut, Dr., D-67127 Roedersheim-Gronau (DE); Minges, Roland, Dr., D-67269 Gruenstadt (DE); Swoboda, Johann, Dr., D-67023 Ludwigshafen (DE); Genz, Manfred, Dr., D-49401 Damme (DE); Scholz, Guenter, Dr., D-49448 Lemfoerde (DE); Streu, Joachim, Dr., D-85221 Dachau (DE)

(56) Entgegenhaltungen:
- DE-A- 1 494 009
- GB-A- 1 083 410
- US-A- 3 502 722

## Beschreibung

Erfindungsgegenstände sind neue 4,4'-disubstituierte Bis-(2,6-diisopropyl-phenyl)-carbodiimide der Formel (I) 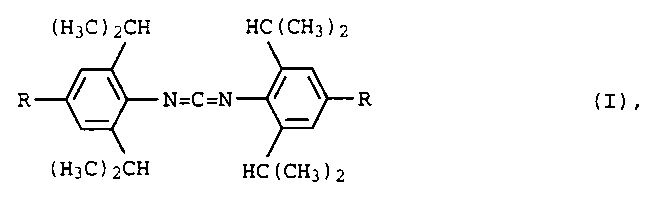
in der R einen 1-Methyl-1-phenyl-ethyl-, einen Phenoxy- oder einen tert.-Butylrest bedeutet, ein Verfahren zu ihrer Herstellung und ihre Verwendung als Stabilisator gegen den hydrolytischen Abbau von Estergruppen enthaltenden Polyadditionsprodukten und Polykondensationsprodukten sowie die zu ihrer Herstellung verwendbaren neuen Monoisocyanate der Formel (II) 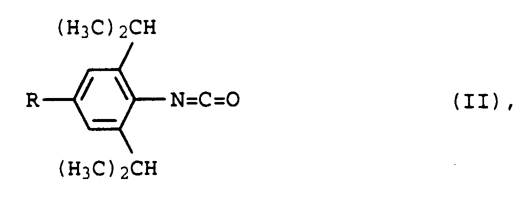
in der R die vorgenannte Bedeutung besitzt.

Organische Carbodiimide sind bekannt. Ihre Chemie wird z.B. beschrieben in Chemical Reviews, Vol. 53 (1953), Seiten 145 bis 166 und Angewandte Chemie 74 (1962), Seiten 801 bis 806.

Monocarbodiimide und oligomere Polycarbodiimide können beispielsweise hergestellt werden durch Einwirkung von basischen Katalysatoren auf sterisch gehinderte Mono- oder Polyisocyanate unter Kohlendioxidabspaltung. Als basische Katalysatoren geeignet sind z.B. gemäß GB-A-1 083 410 heterocyclische, Phosphor gebunden enthaltende Verbindungen und nach Angaben der DE-B-1 130 594 (GB-A-851 936) Phospholene und Phospholidine sowie deren Oxide und Sulfide.

Beschrieben werden ferner Polycarbodiimide mit endständigen Urethangruppen z.B. in der US-A-2 941 983 und DE-B-22 48 751 (US-A-4 076 945). Die Produkte können beispielsweise hergestellt werden durch Carbodiimidisierung von Diisocyanaten mit sterisch gehinderten Isocyanatgruppen und anschließende partielle oder vollständige Urethanisierung der endständigen NCO-Gruppen mit Alkoholen. Bei Verwendung von aromatischen Diisocyanaten mit Isocyanatgruppen unterschiedlicher Reaktivität können die Isocyanatgruppen mit höherer Reaktivität zunächst mit Alkohol partiell oder vollständig in die entsprechenden Urethangruppen übergeführt und danach die verbliebenen Isocyanatgruppen unter Kohlendioxidabspaltung zu Carbodiimidgruppen umgesetzt werden. Oligomere Carbodiimide mit einem mittleren Kondensationsgrad von 2 bis 30, die erhältlich sind durch Oligokondensation von 2,4'-Diisocyanato-diphenylmethan oder eines 3,3',5,5'-Tetra-C₁- bis -C₄-alkyl-4,4'-diisocyanato-diphenylmethans oder von Mischungen dieser gegebenenfalls alkylsubstituierten Diisocyanato-diphenylmethane mit weiteren zwei- oder mehrfunktionellen aromatischen Isocyanate und gewünschtenfalls vollständige oder teilweise Umsetzung der noch freien Isocyanatgruppen der erhaltenen oligomeren Carbodiimide mit einem aliphatischen, araliphatischen oder cycloaliphatischen Alkohol oder Amin werden in der DE-A-41 26 359 beschrieben.

Die Carbodiimide finden vorzugsweise Verwendung als Stabilisatoren gegen die hydrolytische Spaltung von Kunststoffen auf Polyesterbasis. Nach Angaben der DE-A-1 494 009 (US-A-3 193 523) eignen sich hierfür insbesondere aromatische und/oder cycloaliphatische Monocarbodiimide, die in 2- und 2'-Stellung substituiert sind, wie z.B. 2,2',6,6'-Tretraisopropyl-diphenylcarbodiimid. Polycarbodiimide mit einem Molekulargewicht von über 500 und einem Gehalt von mehr als 3 Carbodiimidgruppen werden als Stabilisatoren gegen Einflüsse von Wärme und Feuchtigkeit in estergruppenhaltigen Kunststoffen in der DE-B-1 285 747 (US-A-3 193 522) beschrieben. Obgleich durch den Zusatz dieser (Poly)carbodiimide als Stabilisatoren eine weitgehende Stabilität Estergruppen haltiger Kunststoffe gegen feuchte Wärme, Wasser und Wasserdampf erzielt werden kann, weisen die Produkte auch Nachteile auf. Nachteilig an den technisch vorzugsweise verwendeten tetra-alkylsubstituierten Monocarbodiimiden, wie z.B. 2,2',6,6'-Tetraisopropyldiphenyl-carbodiimid, ist ihr relativ hoher Dampfdruck und bedingt durch das niedrige Molekulargewicht ihre Neigung zur Migration aus den Polyadditionsprodukten z.B. thermoplastischen Polyurethanen (TPU), oder Polykondensationsprodukten, z.B. Polyterephthalaten. Zur Beseitigung diese Mangels finden nach Angaben der EP-A-0 460 481 (CA-A-2 043 820) substituierte Monocarbodiimide oder oligomere, substituierte Polycarbodiimide mit endständigen Isocyanatgruppen Verwendung, die hergestellt werden aus substituierten Diisocyanaten und die praktisch weder in der Hitze, z.B. unter den üblichen Verarbeitungsbedingungen, noch bei Raumtemperatur giftige, flüchtige aus den eingesetzten Carbodiimiden stammende Substanzen abspalten. Polycarbodiimide dieser Art besitzen höhere Schmelzpunkte oder sind unschmelzbar und können nur mit einem beträchtlichen apparativen und zeitlichen Aufwand in die Polyurethane und/oder ihre Ausgangsstoffe eingebracht werden. Die Verteilung der Polycarbodiimide in den Estergruppen enthaltenden Kunststoffen ist daher vielfach unzureichend homogen, so daß die Stabilisatorwirksamkeit nicht den Erwartungen entspricht. Durch die Überführung eines Teils der endständigen Isocyanat- in Urethangruppen, z.B. gemäß DE-A-22 48 751 oder US-A-2 941 983, können zwar leichter schmelzende Polycarbodiimidderivate erhalten werden. Diese zusätzliche Reaktionsstufe verteuert jedoch nicht nur die Produkte, sondern die damit verbundene Molekulargewichtserhöhung führt auch zu einer Reduzierung des Anteils an wirksamen Carbodiimidgruppen in einer vergleichbaren Mengeneinheit.

Die Aufgabe der vorliegenden Erfindung bestand darin, die vorgenannten Nachteile ganz oder zumindest teilweise zu beseitigen und Hydrolyseschutzmittel zur Verfügung zu stellen, die homogen in den Estergruppen enthaltenden Kunststoffen, vorzugsweise Polyurethanen und insbesondere TPU, löslich sind, ohne daß es hierzu zusätzlicher Homogenisierungsschritte bedarf.

Diese Aufgabe konnte überraschenderweise gelöst werden durch die Verwendung von ausgewählten trisubstituierten Phenylisocyanaten zur Herstellung der Carbodiimide.

Gegenstand der Erfindung sind somit in 4,4'-Stellung disubstituierte Bis-(2,6-diisopropyl-phenyl)-carbodiimide der Formel (I) 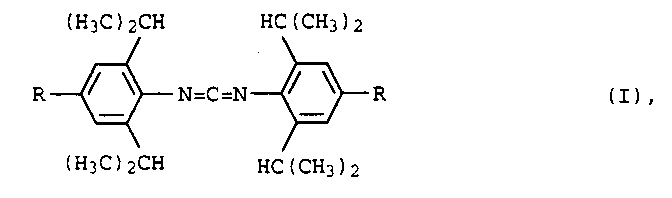
in der R einen 1-Methyl-1-phenyl-ethyl-, einen Phenoxy- oder tert.-Butylrest bedeutet.

Monocarbodiimide mit einer Struktur der Formel (I) sind 4,4'-Di-(1-methyl-1-phenyl-ethyl)-2,2',6,6'-tetraisopropyl-diphenylcarbodiimid der Formel 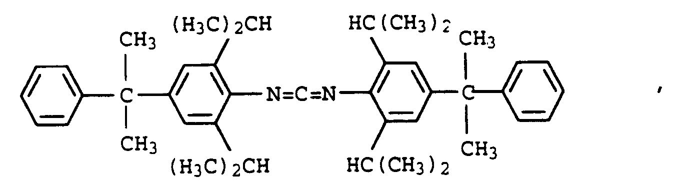
4,4'-Diphenoxy-2,2',6,6'-tetraisopropyl-diphenylcarbodiimid der Formel 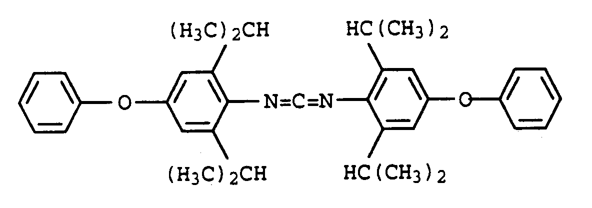
und
4,4'-Di-tert.-butyl-2,2',6,6'-tetraisopropyl-diphenylcarbodiimid der Formel 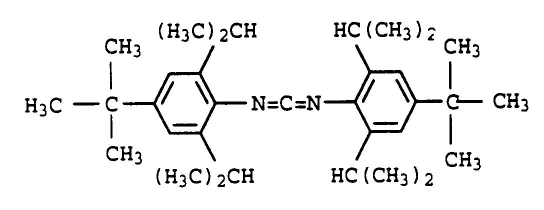
Gegenstände der Erfindung sind ferner ein Verfahren zur Herstellung der 4,4'-disubstituierten Bis-(2,6-diisopropyl-phenyl)-carbodiimide der Formel (I) durch Kondensation von in 4-Stellung substituierten 2,6-Diisopropyl-phenylisocyanaten in Gegenwart von Katalysatoren, das dadurch gekennzeichnet ist, daß die in 4-Stellung substituierten 2,6-Diisopropyl-phenylisocyanate eine Struktur der Formel (II) 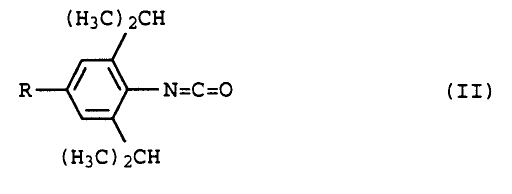
besitzen, in der R einen 1-Methyl-1-phenyl-ethyl-, einen Phenoxy- oder tert.-Butylrest bedeutet und die Verwendung der in 4,4'-Stellung disubstituierten Bis-(2,6-diisopropyl-phenyl)-carbodiimide der Formel (I) als Stabilisatoren gegen den hydrolytischen Abbau von Estergruppen gebunden enthaltenden Polyadditions- oder Polykondensationsprodukte sowie die zur Herstellung der erfindungsgemäßen 4,4'-disubstituierten Bis-(2,6-diisopropyl-phenyl)-carbodiimide der Formel (I) verwendbaren in 4-Stellung substituierten 2,6-Diisopropyl-phenylisocyanate der Formel (II) 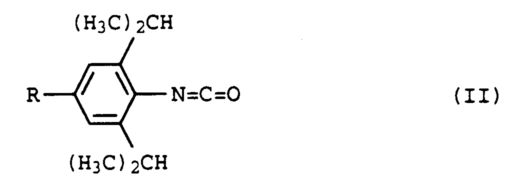
in der R einen 1-Methyl-1-phenyl-ethyl-, einen Phenoxy- oder tert.-Butylrest bedeutet.

Die erfindungsgemäßen neuen 4,4'-disubstituierten Bis-(2,6-diisopropyl-phenyl)-carbodiimide besitzen eine mit den technisch genutzten Carbodiimiden zumindest vergleichbare hohe Hydrolyseschutzwirkung und können unter Beachtung der Arbeitschutzvorschriften problemlos ohne zusätzliche Homogenisierungsschritte wirtschaftlich dosiert und in die Estergruppen enthaltende Kunststoffe, vorzugsweise Polyurethane, eingebracht werden. Die erfindungsgemäßen Carbodiimide besitzen einen vergleichsweise geringen Dampfdruck und zeigen ein vernachlässigbares Migrationsverhalten. Erwähnenswert ist ferner, daß unerwünschte Nebenreaktionen, wie sie z.B. bei der Polycarbodiimidherstellung aus Diisocyanaten durch deren unerwünschte Cyclisierung oder Polymerisation auftreten können, durch die Verwendung von in 4-Stellung substituierten 2,6-Diisopropyl-phenylisocyanaten vermieden werden.

Die den erfindungsgemäßen Carbodiimiden zugrundeliegenden in 4-Stellung substituierten 2,6-Diisopropyl-phenylisocyanate der Formel (II) 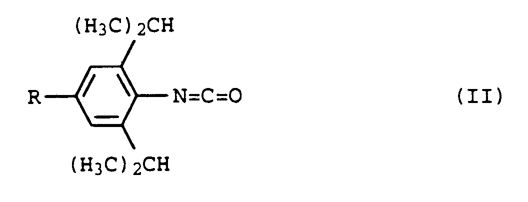
in der R einen 1-Methyl-1-phenyl-ethyl-, einen Phenoxy- oder tert.-Butylrest bedeutet, sind neu. Die genannten neuen Isocyanate weisen folgende Strukturformeln auf 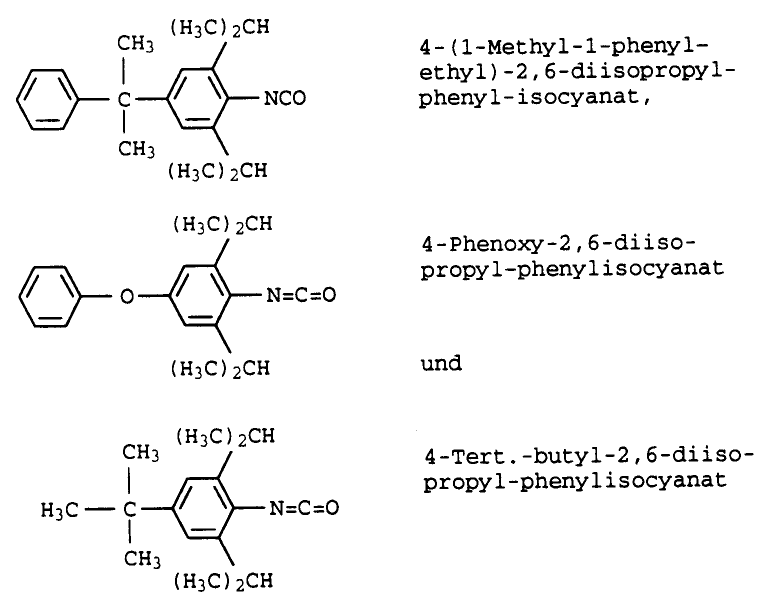
Die in 4-Stellung substituierten 2,6-Diisopropyl-phenylisocyanate können hergestellt werden nach bekannten Verfahren z.B. durch Umsetzung der entsprechend in 4-Stellung substituierten 2,6-Diisopropyl-aniline mit Phosgen zweckmäßigerweise in Gegenwart von unter den Reaktionsbedingungen inerten Lösungsmitteln bei Temperaturen von üblicherweise unter 100°C, vorzugsweise unter 50°C zu Carbamidsäurechloriden der Formel (III)) 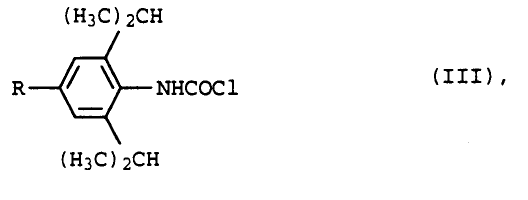
in der R die vorgenannte Bedeutung besitzt, und anschließende thermische Spaltung der erhaltenen in 4-Stellung substituierten 2,6-Diisopropyl-phenylcarbamidsäurechloride bei Temperaturen von üblicherweise über 100°C, vorzugsweise von 110 bis 160°C in die erfindungsgemäßen in 4-Stellung substituierten 2,6-Diisopropyl-phenylisocyanate oder durch Umsetzung der in 4-Stellung substituierten 2,6-Diisopropyl-aniline mit Harnstoff in Gegenwart von Alkoholen und/oder Carbamidsäureestern in Gegenwart oder Abwesenheit von Alkoholen bei Temperaturen von üblicherweise 100 bis 200°C, vorzugsweise 125 bis 175°C zu in 4-Stellung substituierten 2,6-Diisopropyl-phenylcarbamidsäureestern der Formel (IV) 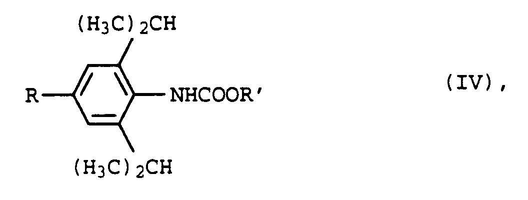
in der R die vorgenannte Bedeutung besitzt und R' ein linearer oder verzweigter Alkylrest mit 1 bis 12 Kohlenstoffatomen, ein Cyclohexyl- oder ein Phenylrest ist, und anschließende thermische Spaltung der in 4-Stellung substituierten 2,6-Diisopropyl-phenyl-carbamidsäureester in der Gas- oder Flüssigphase bei Temperaturen über 150°C in die erfindungsgemäßen in 4-Stellung substituierten 2,6-Diisopropyl-phenylisocyanate.

Zur Herstellung der erfindungsgemäßen in 4,4'-Stellung disubstituierten Bis-(2,6-diisopropyl-phenyl)-carbodiimide können die obengenannten in 4-Stellung substituierten 2,6-Diisopropyl-phenylisocyanate bei erhöhten Temperatun, z.B. bei Temperaturen von 50 bis 200°C, vorzugsweise von 130 bis 175°C, zweckmäßigerweise in Gegenwart von Katalysatoren unter Kohlendioxidabspaltung kondensiert werden. Hierfür geeignete Verfahren werden beispielsweise beschrieben in der GB-A-1 083 410, der DE-B 1 130 594 (GB-A-851 936) und der DE-A-11 56 401 (US-A-3 502 722). Als Katalysatoren vorzüglich bewährt haben sich z.B. Phosphorverbindungen, die vorzugsweise ausgewählt werden aus der Gruppe der Phospholene, Phospholenoxide, Phospholidine und Phospholidinoxide. Wenn die Reaktionsmischung einen Gehalt an NCO-Gruppen von kleiner als 1 Gew.-% besitzt, wird die Carbodiimidbildung üblicherweise beendet. Hierzu können die Katalysatoren unter vermindertem Druck abdestilliert oder durch Zusatz eines Desaktivators, wie z.B. Phosphortrichlorid, desaktiviert werden. Die Carbodiimidherstellung kann ferner in Gegenwart oder Abwesenheit von Lösungsmitteln durchgeführt werden.

Die erfindungsgemäßen Monocarbodiimide eignen sich hervorragend als Akzeptor für Carboxylverbindungen und finden daher vorzugsweise Verwendung als Stabilisatoren gegen den hydrolytischen Abbau von Estergruppen gebunden enthaltende Polykonensationsprodukte z.B. Polyester, Polyetherester, Polyesteramide und Polycaprolactone, und Polyadditionsprodukte, z.B. Polyurethane, Polyharnstoffe und Polyurethan-Polyharnstoff-Elastomere. Aufgrund ihrer guten Löslichkeit in den Aufbaukomponenten zur Herstellung von Polyurethanen und in den gebildeten Polyurethanen eignen sich die Monocarbodiimide insbesondere als Stabilisatoren gegen den hydrolytischen Abbau von Polyurethanen, vorzugsweise kompakten oder zelligen Polyurethan-Elastomeren und insbesondere TPU.

Die Konzentration der erfindungsgemäßen Monocarbodiimide in den zu stabilisierenden Estergruppen enthaltenden Polykondensations- oder Polyadditionsprodukte beträgt im allgemeinen 0,05 bis 10 Gew.-%, vorzugsweise 0,1 bis 5 Gew.-%. In Einzelfällen kann, je nach der Beanspruchung des Kunststoffs durch Hydrolyse, die Konzentration auch höher sein.

Die erfindungsgemäß verwendbaren Monocarbodiimide können nach verschiedenen Methoden in die zu stabilisierende Estergruppen enthaltende Polyadditions- oder Polykondensationsprodukte eingebracht werden. Beispielsweise können die erfindungsgemäßen Monocarbodiimide mit einer der Aufbaukomponenten zur Herstellung der Polyadditionsprodukte, z.B. den Polyisocyanaten oder/und Polyhydroxylverbindungen zur Herstellung von Polyurethanen gemischt werden oder die Monocarbodiimide können der Reaktionsmischung zur Herstellung der Polyurethane zudosiert werden. Nach einer anderen Verfahrensweise können die erfindungsgemäßen Monocarbodiimide der Schmelze der ausreagierten Polyadditions- oder Polykondensationsprodukte einverleibt werden. Es ist jedoch auch möglich Granulate der Polyadditions- oder Polykondensationsprodukte mit den erfindungsgemäßen Monocarbodiimiden zu beschichten und diese bei einer nachfolgenden Herstellung von Formkörpern durch Schmelzextrusion in die Kunststoffmassen einzubringen. Zur Herstellung von Polyurethan-Gießelastomeren und TPU auf Polyester-polyolbasis werden nach einer bevorzugten Ausführungsform zunächst die carboxylgruppenhaltigen Polyester-polyole zur Reduzierung der Säuregehalte mit den erfindungsgemäßen Monocarbodiimiden behandelt und danach diese, gegebenenfalls unter Zugabe von weiteren Mengen an Monocarbodiimiden, mit Polyisocyanaten, gegebenenfalls in Gegenwart zusätzlicher Hilfsmittel und Additive, zur Reaktion gebracht.

### Beispiele

### Herstellung der 4-substituierten 2,6-Diisopropyl-phenylisocyanate

### Beispiel 1

In einer 6000 ml Laborphosgenierungsapparatur, die bestand aus einem Rührgefäß mit Rührer, einem Rückflußkühler mit einer Gasableitungsvorrichtung, einem Tropftrichter und einer Mischdüse, wurde eine Lösung aus 99 g (1 mol) Phosgen in 1300 ml Monochlorbenzol vorgelegt und in diese bei einer Temperatur unter 50°C und unter intensivem Rühren eine Lösung aus 252 g (0,85 mol) 4-(1-Methyl-1-phenyl-ethyl)-2,6-diisopropyl-anilin in 1300 ml Monochlorbenzol durch eine mit Stickstoff gespülte Düse zugeführt. Ferner ließ man in einem Zeitraum von einer Stunde 198 g (2 Mol) Phosgen zutropfen.

Unter den genannten Reaktionsbedingungen, der sogenannten Kaltphosgenierung wurde keine Ausfällung von gebildetem 4-(1-Methyl-1-phenyl-ethyl)-2,6-diisopropyl-anilin-carbamidsäurechlorid-oder -hydrochlorid beobachtet.

Nach vollständiger Eindüsung der Aminlösung wurde die Phosgenzugabe beendet und die hellorangene Reaktionslösung im Verlaufe von 1,75 Stunden unter Rückflußkühlung auf 125°C erhitzt. Aus der Reaktionsmischung wurde danach das überschüssige Phosgen und das Monochlorbenzol abdestilliert und das gebildete rohe Isocyanat bei 190°C, 10 mbar eine Stunde lang entchloriert.

Man erhielt 272,3 g eines hellbraunen Rohprodukts mit einem NCO-Gehalt von 12,9 Gew.-% (berechnet 13,1 Gew.-%) mit einer durch Gaschromatographie bestimmten Reinheit von 95,8 Gew.-%, einem Gehalt an hydrolysierbarem Chlor von 111 ppm und einer Viskosität, gemessen bei 25°C nach Ubbelohde, von 75 mPas.

Das erhaltene Rohprodukt wurde durch Destillation unter vermindertem Druck gereinigt. Man erhielt 4-(1-Methyl-1-phenylethyl)-2,6-diisopropyl-phenylisocyanat als eine bei 135°C/0,5 mbar siedende, leicht milchig trübe Flüssigkeit in einer Ausbeute von 98,6 % der Theorie. Die Struktur wurde durch ¹H-NMR- und IR-Spektroskopie nachgewiesen. Die Elementaranalyse ergab folgende Werte:

| | | | | |
|---|---|---|---|---|
| C: | berechnet: | 82,2 Gew.-%; | gefunden: | 81,9 Gew.-% |
| H: | berechnet: | 8,5 Gew.-%; | gefunden: | 8,6 Gew.-% |
| N. | berechnet: | 4,4 Gew.-%; | gefunden: | 4,8 Gew.-% |

### Beispiel 2

Man verfuhr analog den Angaben des Beispiels 1, verwendete jedoch als Amin 229 g (0,85 mol) 4-Phenoxy-2,6-diisopropyl-anilin und führte keine Entchlorierung bei 190°C/10 mbar durch.

Man erhielt 274,9 g eines dunkelbraunen Rohprodukts mit einem NCO-Gehalt von 14,0 Gew.-% (berechnet 14,2 Gew.-%), mit einer durch Gaschromatographie bestimmten Reinheit von 98,3 Gew.-%, einem Gehalt an hydrolysierbarem Chlor von 600 ppm und einer Viskosität, gemessen bei 25°C nach Ubbelohde, von 65 mPas.

Das erhaltene Rohprodukt wurde durch Destillation unter vermindertem Druck gereinigte. Man erhielt 4-Phenoxy-2,6-diisopropyl-phenylisocyanat als eine bei 134°C/0,5 mbar siedende klare, farblose Flüssigkeit in einer Ausbeute von 97,8 % der Theorie.

Die Struktur wurde durch ¹H-NMR- und IR-Spektroskopie nachgewiesen. Die Elementaranalyse ergab folgende Werte:

| | | | | |
|---|---|---|---|---|
| C: | berechnet: | 77,3 Gew.-%; | gefunden: | 77,1 Gew.-% |
| H: | berechnet: | 7,2 Gew.-%; | gefunden: | 7,3 Gew.-% |
| N. | berechnet: | 4,7 Gew.-%; | gefunden: | 5,1 Gew.-% |

### Beispiel 3

Man verfuhr analog den Angaben des Beispiels 1, verwendete jedoch als Amin 198 g (0,85 mol) 4-tert.-Butyl-2,6-diisopropyl-anilin.

Man erhielt 222 g eines hellbraunen Rohprodukts mit einem NCO-Gehalt von 16,1 Gew.-% (berechnet 16,2 Gew.-%), mit einer durch Gaschromatographie bestimmten Reinheit von 97,8 Gew.-%, einem Gehalt an hydrolysierbarem Chlor von 127 ppm und einer Viskosität, gemessen bei 25°C nach Ubbelohde, von 40 mPas.

Das erhaltene Rohprodukt wurde durch Destillation unter vermindertem Druck gereinigt. Man erhielt 4-tert.-Butyl-2,6-diisopropyl-phenylisocyanat als eine bei 86°C/0,5 mbar siedende klare, farblose Flüssigkeit in einer Ausbeute von 98,2 % der Theorie.

Die Struktur wurde durch ¹H-NMR- und IR-Spektroskopie nachgewiesen. Die Elementaranalyse ergab folgende Werte:

| | | | | |
|---|---|---|---|---|
| C: | berechnet: | 78,7 Gew.-%; | gefunden: | 78,4 Gew.-% |
| H: | berechnet: | 9,7 Gew.-%; | gefunden: | 9,7 Gew.-% |
| N: | berechnet: | 5,4 Gew.-%; | gefunden: | 5,7 Gew.-% |

### Herstellung der 4,4'-disubstituierten-(2,6-diisopropyl-phenyl)-carbodiimide

### Beispiel 4

100 Gew.-Teile nach Beispiel 1 hergestelltes 4-(1-Methyl-1-phenyl-ethyl)-2,6-diisopropyl-phenylisocyanat wurde in Gegenwart von 0,1 Gew.-Teilen 1-Methyl-2-phospholen-1-oxid lösungsmittelfrei auf 160 bis 170°C erhitzt und in diesem Temperaturbereich unter starker Kohlendioxidentwicklung kondensiert. Nach Erreichen eines NCO-Gehalts des Reaktionsgemisches von kleiner als 1 Gew.-%, hierzu war eine Reaktionszeit von ungefähr 14 Stunden erforderlich, wurden der zugesetzte Katalysator und Reste von nicht umgesetzten 4-(1-Methyl-1-phenyl-ethyl)-2,6-diisopropyl-phenylisocyanat bei einer Temperatur von 180°C und einem Druck von 0,1 mbar abdestilliert. Man erhielt 92,1 Gew.-Teile Bis-(4-(1-methyl-1-phenyl-ethyl)-2,6-diisopropyl-phenyl)-carbodiimid mit einem NCO-Gehalt von kleiner als 0,4 Gew.-%, einem Gehalt an -N=C=N-Gruppen von 65 mg/g und einen Schmelzpunkt von 59°C. Das entspricht einer Ausbeute von 98,9 % der Theorie.

Seine Struktur wurde durch ¹H-NMR- und IR-Spektrum nachgewiesen.

Die Elementaranalyse erbrachte folgende Werte:

| | | | | |
|---|---|---|---|---|
| C: | berechnet: | 86,2 Gew.-%; | gefunden: | 85,9 Gew.-% |
| H: | berechnet: | 9,1 Gew.-%; | gefunden: | 8,9 Gew.-% |
| N. | berechnet: | 4,7 Gew.-%; | gefunden: | 4,9 Gew.-% |

### Beispiel 5

Man verfuhr analog den Angaben des Beispiels 4, verwendete jedoch als Isocyanat 100 Gew.-Teile des nach Beispiel 2 hergestellten 4-Phenoxy-2,6-diisopropyl-phenylisocyanats.

Man erhielt als Destillationsrückstand 90.7 Gew.-Teile Bis-(4-phenoxy-2,6-diisopropyl-phenyl)-carbodiimid mit einem NCO-Gehalt von kleiner als 0,4 Gew.-%, einem Gehalt an -N=C=N-Gruppen von 71 mg/g und einen Schmelzpunkt von 70°C. Das entspricht einer Ausbeute von 98,1 % der Theorie.

Seine Struktur wurde durch ¹H-NMR- und IR-Spektrum nachgewiesen.

Die Elementaranalyse erbrachte folgende Werte:

| | | | | |
|---|---|---|---|---|
| C: | berechnet: | 81,3 Gew.-%; | gefunden: | 81,0 Gew.-% |
| H: | berechnet: | 7,7 Gew.-%; | gefunden: | 7,5 Gew.-% |
| N: | berechnet: | 5,1 Gew.-%; | gefunden: | 5,4 Gew.-% |

### Beispiel 6

Man verfuhr analog den Angaben des Beispiels 4, verwendete jedoch als Isocyanat 100 Gew.-Teile des nach Beispiel 3 hergestellten 4-tert.-Butyl-2,6-diisopropyl-phenylisocyanats.

Man erhielt als Destillationsrückstand 90,1 Gew.-Teile Bis-(4-tert.-butyl-2,6-diisopropyl-phenyl)-carbodiimid mit einem NCO-Gehalt von kleiner als 0,4 Gew.-%, einem Gehalt an -N=C=N-Gruppen von 81 mg/g und einen Schmelzpunkt von 80°C. Das entspricht einer Ausbeute von 98,5 % der Theorie.

Seine Struktur wurde durch ¹H-NMR- und IR-Spektrum nachgewiesen.

Die Elementaranalyse erbrachte folgende Werte:

| | | | | |
|---|---|---|---|---|
| C: | berechnet: | 83,5 Gew.-%; | gefunden: | 83,2 Gew.-% |
| H: | berechnet: | 10,6 Gew.-%; | gefunden: | 10,4 Gew.-% |
| N: | berechnet: | 5,9 Gew.-%; | gefunden: | 6,1 Gew.-% |

### Herstellung von mit Carbodiimiden stabilisierte thermoplastische Polyurethane (TPU)

### Beispiele 7 bis 13 und Vergleichsbeispiel I bis III

Eine Mischung aus 1000 Gew.-Teilen (0,5 mol) eines 1,4-Butandiol-1,6-hexandiol-polyadipats mit einer Hydroxylzahl von 56 und n Gew.-% eines der nachfolgend genannten Carbodiimide wurde eine Stunde lang bei 110°C und einem Druck von 2 mbar getrocknet.

Nachdem der Mischung 113 Gew.-Teile (1,26 mol) Butandiol-1,4 einverleibt wurden, wurde diese auf 70°C erwärmt und unter intensivem Rühren mit 440 Gew.-Teilen einer auf 65°C erhitzten Schmelze aus 4,4'-Diphenylmethan-diisocyanat versetzt.

Nach Erreichen einer Reaktionstemperatur von 120°C wurde die homogene Reaktionsmischung auf eine auf 125°C temperierte Metallplatte gegossen. Nach ungefähr 2 Minuten wurde das heiße Rohprodukt von der Metallplatte genommen, grob zerkleinert und danach 15 Stunden bei 100°C getempert. Das erhaltene TPU, das eine Shore-Härte 85 A besaß, wurde abgekühlt, granuliert und danach zu Prüfkörpern verspritzt.

Zur Durchführung eines standardisierten Hydrolysetests wurden die Prüfkörper in vollentsalztem Wasser bei 80°C gelagert und nach 42 Tagen erneut die Zugfestigkeit und die Bruchdehnung nach DIN 53 504 gemessen.

Die verwendeten Bis-(4-substituierten-2,6-diisopropyl-phenyl)-carbodiimide und die als Vergleichsprodukte eingesetzten Carbodiimide und ihre Mengen in Gew.-% sowie die an den Prüfkörpern gemessene Zugfestigkeit und Dehnung sind in der nachfolgenden Tabelle zusammengefaßt.

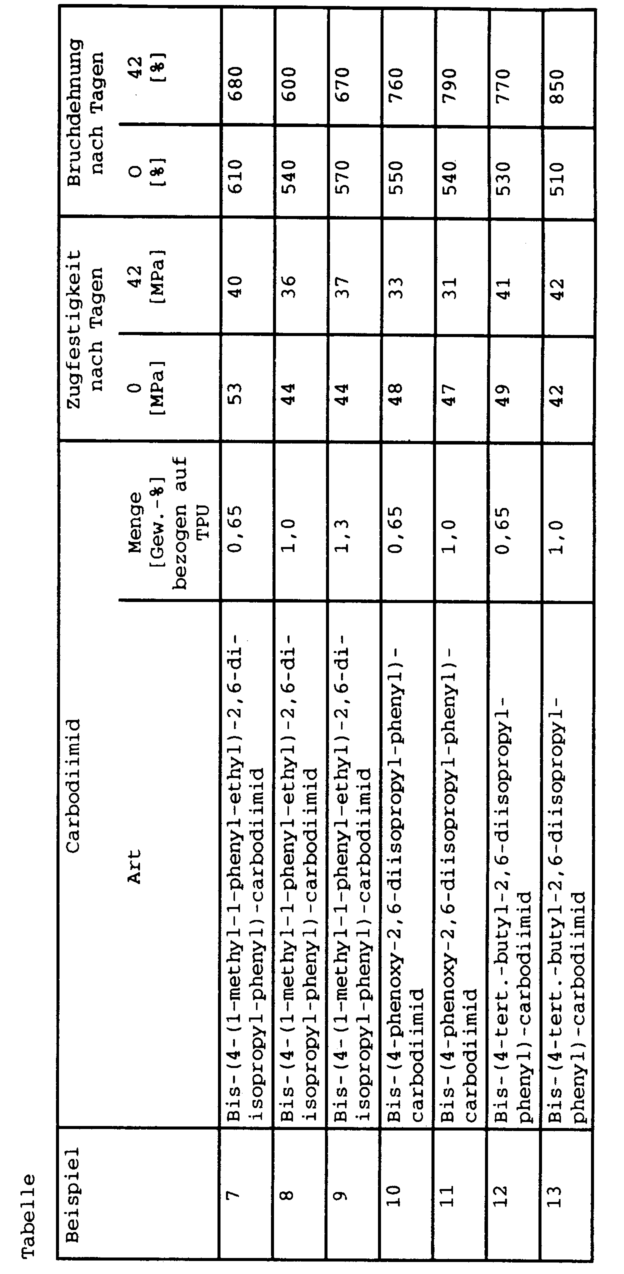

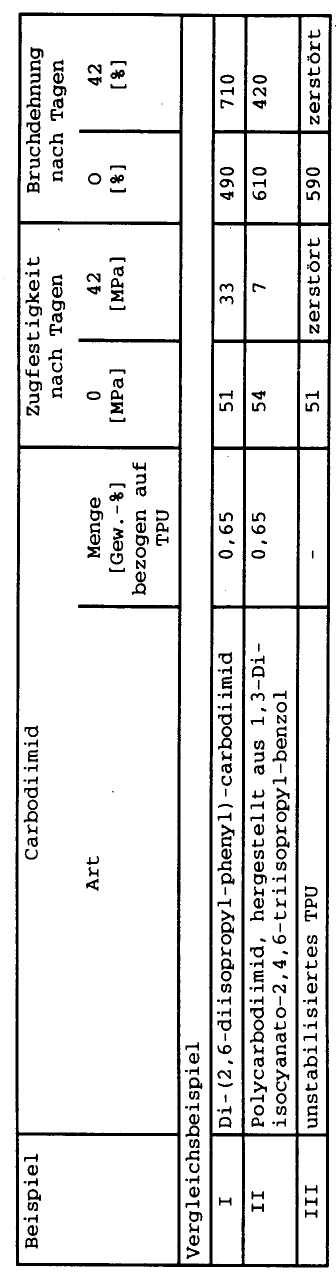

## Patentansprüche

1. In 4,4'-Stellung disubstituierte Bis-(2,6-diisopropyl-phenyl)-carbodiimide der Formel 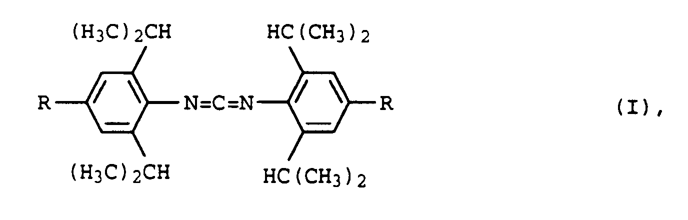 in der R einen 1-Methyl-1-phenyl-ethyl-, einen Phenoxy- oder tert.-Butylrest bedeutet.

2. Monocarbodiimid der Formel

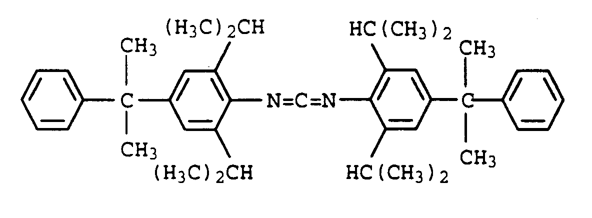

3. Monocarbodiimid der Formel

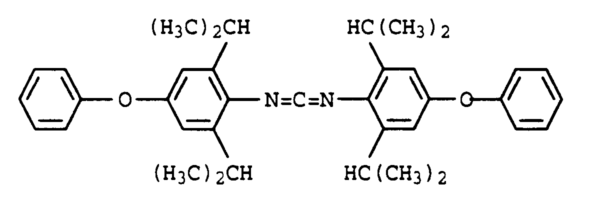

4. Monocarbodiimid der Formel

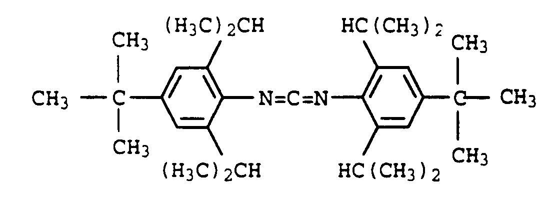

5. Verfahren zur Herstellung von 4,4'-disubstituierten Bis-(2,6-diisopropyl-phenyl)-carbodiimiden durch Kondensation von in 4-Stellung substituierten 2,6-Diisopropyl-phenylisocyanaten in Gegenwart von Katalysatoren, dadurch gekennzeichnet, daß die in 4-Stellung substituierten 2,6-Diisopropyl-phenylisocyanate eine Struktur der Formel II 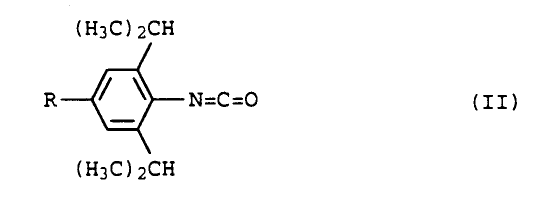 besitzen, in der R einen 1-Methyl-1-phenyl-ethyl-, einen Phenoxy- oder tert.-Butylrest bedeutet.

6. Verfahren nach Anspruch 5, dadurch gekennzeichnet, daß man als Katalysator Phosphorverbindungen, ausgewählt aus der Gruppe der Phospholene, Phospholenoxide, Phospholidine und Phospholidinoxide verwendet.

7. Verwendung der in 4,4'-Stellung disubstituierten Bis-(2,6-diisopropyl-phenyl)-carbodiimide der Formel (I) 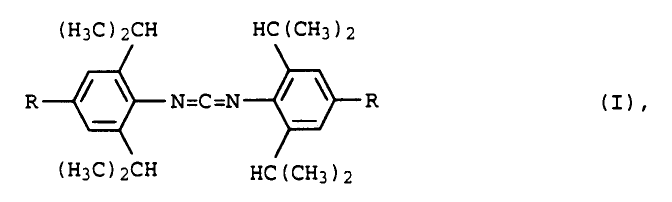 in der R einen 1-Methyl-1-phenyl-ethyl-, einen Phenoxy- oder tert.-Butylrest bedeutet, als Stabilisatoren gegen den hydrolytischen Abbau von Estergruppen gebunden enthaltenden Polyadditions- oder Polykondensationsprodukte.

8. Verwendung der in 4,4'-Stellung disubstituierten Bis-(2,6-diisopropyl-phenyl)-carbodiimide der Formel (I) 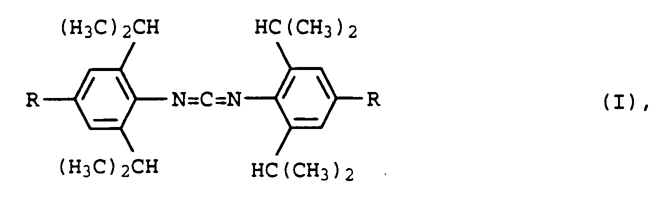 in der R einen 1-Methyl-1-phenyl-ethyl-, einen Phenoxy- oder tert.-Butylrest bedeutet, als Stabilisatoren gegen den hydrolytischen Abbau von Polyurethanen.

9. In 4-Stellung substituierte 2,6-Diisopropyl-phenylisocyanate der Formel (II) 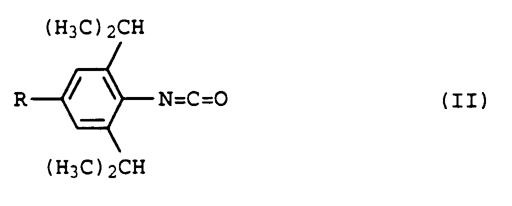 in der R einen 1-Methyl-1-phenyl-ethyl-, einen Phenoxy- oder tert.-Butylrest bedeutet.

## Claims

1. A 4,4'-disubstituted bis(2,6-diisopropylphenyl)-carbodiimide of the formula 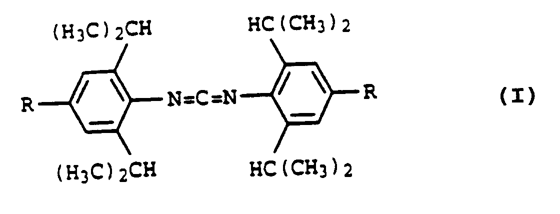 where R is 1-methyl-1-phenylethyl, phenoxy or tert-butyl.

2. The monocarbodiimide of the formula

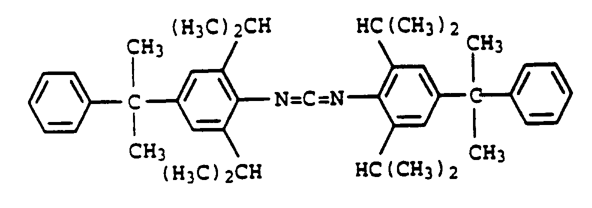

3. The monocarbodiimide of the formula

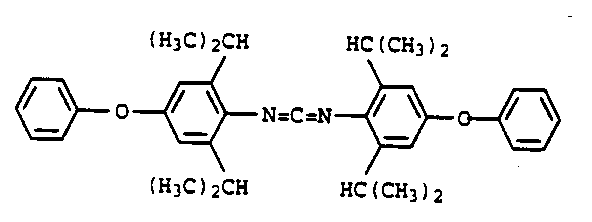

4. The monocarbodiimide of the formula

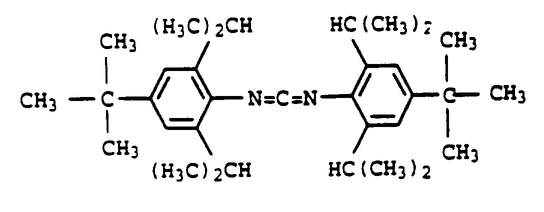

5. A process for the preparation of 4,4'-disubstituted bis(2,6-diisopropylphenyl)carbodiimides by condensing 4-substituted 2,6-diisopropylphenyl isocyanates in the presence of catalysts, wherein the 4-substituted 2,6-diisopropylphenyl isocyanates have a structure of the formula II 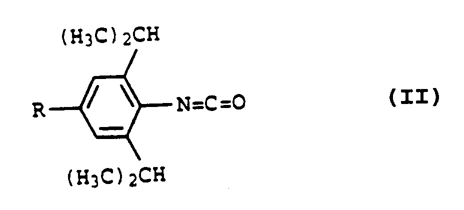 where R is 1-methyl-1-phenylethyl, phenoxy or tert-butyl.

6. A process as claimed in claim 5, wherein the catalyst used is a phosphorus compound selected from the group consisting of phospholenes, phospholene oxides, phospholidines and phospholidine oxides.

7. The use of the 4,4'-disubstituted bis(2,6-diisopropylphenyl)carbodiimides of the formula (I) 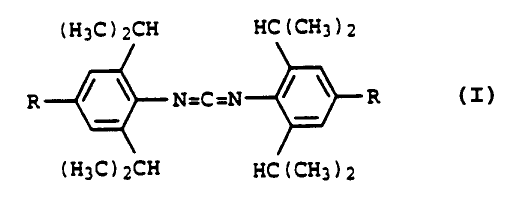 where R is 1-methyl-1-phenylethyl, phenoxy or tert-butyl, as stabilizers against hydrolytic degradation of polyaddition or polycondensation products containing bonded ester groups.

8. The use of the 4,4'-disubstituted bis(2,6-diisopropylphenyl)carbodiimides of the formula (I) 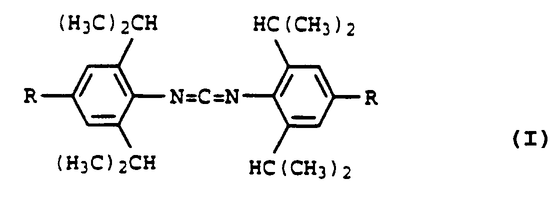 where R is 1-methyl-1-phenylethyl, phenoxy or tert-butyl, as stabilizers against hydrolytic degradation of polyurethanes.

9. A 4-substituted 2,6-diisopropylphenyl isocyanate of the formula (II) 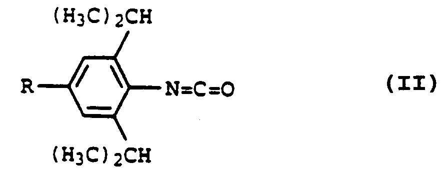 where R is 1-methyl-1-phenylethyl, phenoxy or tert-butyl.

## Revendications

1. Bis-(2,6-diisopropylphényl)-carbodiimide disubstitué en position 4,4' de formule 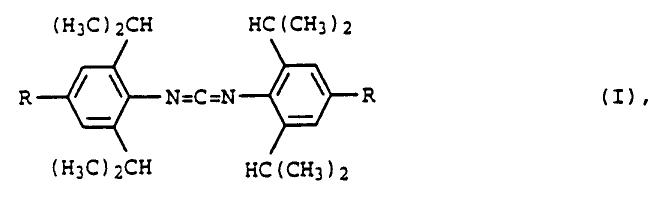 dans laquelle R représente un reste 1-méthyl-1-phényl-éthyle, un reste phénoxy- ou tert-butyle.

2. Monocarbodiimide de formule

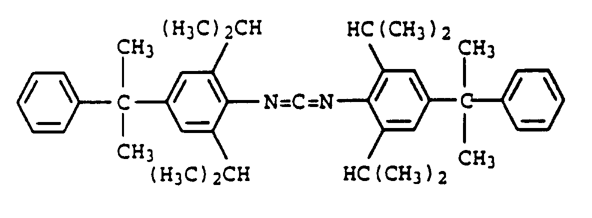

3. Monocarbodiimide de formule

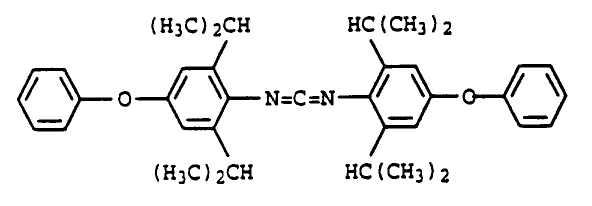

4. Monocarbodiimide de formule

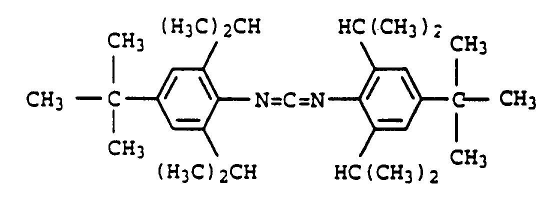

5. Procédé de préparation de bis-(2,6-diisopropylphényl)-carbodiimide disubstitué en position 4,4' par condensation de 2,6-diisopropyl-phénylisocyanates substitués en position 4, en présence de catalyseurs, caractérisé par le fait que le 2,6-diisopropylphénylisocyanate substitué en position 4 possède une structure de formule II 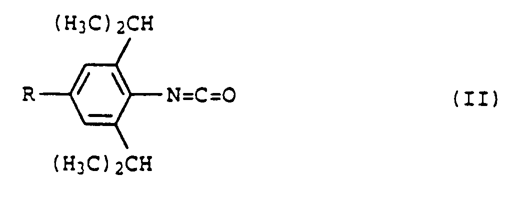 dans laquelle R représente un reste 1-méthyl-1-phényléthyle, un reste phénoxy ou tert-butyle.

6. Procédé selon la revendication 5, caractérisé par le fait qu'on utilise, comme catalyseur, des composés phosphorés, choisis dans le groupe des phospholène, phospholénoxyde, phospholidine et phospholidinoxyde.

7. Utilisation des bis-(2,6-diisopropylphényl)-carbodiimide disubstitué en position 4,4' de formule I 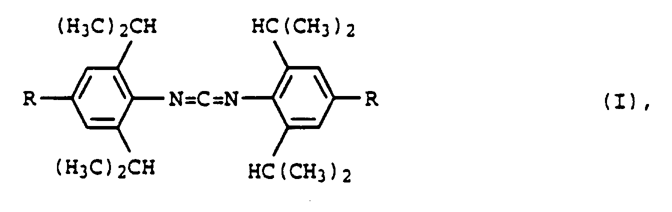 dans laquelle R représente un reste 1-méthyl-1-phényl-éthyle, un reste phénoxy- ou tert-butyle, comme stabilisateurs contre la dégradation hydrolytique des produits de polyaddition ou polycondensation contenant liés des groupes ester.

8. Utilisation des bis-(2,6-diisopropylphényl)-carbodiimide disubstitué en position 4,4' de formule I 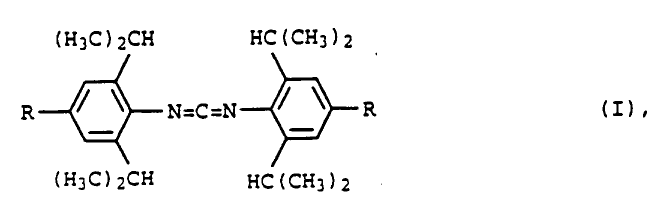 dans laquelle R représente un reste 1-méthyl-1-phényl-éthyle, un reste phénoxy- ou tert-butyle, comme stabilisateurs contre la dégradation hydrolytique des polyuréthanes.

9. 2,6-diisopropyl-phénylisocyanate substitué en position 4 de formule II 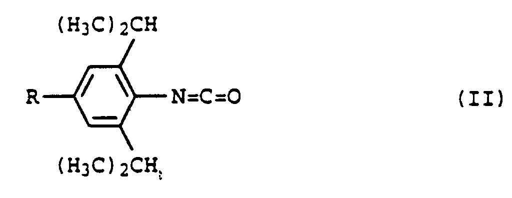 dans laquelle R représente un reste 1-méthyl-1-phényle, un reste phénoxy ou tert-butyle.
